# EUROPEAN PATENT APPLICATION

(11) **EP 1 693 455 A1**
(43) Date of publication of application: **23.08.2006**
(21) Application number: 04818224.0
(22) Date of filing: 08.11.2004
(51) Int. Cl.: C12N 15/12, C07K 14/705, C12N 1/15, C12N 1/19, C12N 1/21, C12N 5/10, C12Q 1/02, C12Q 1/68

(54) **SIMIAN ORL1 GENE AND METHOD OF ASSESSING COMPOUND**

(30) Priority: 06.11.2003 JP 2003377006
(71) Applicant: BANYU PHARMACEUTICAL CO., LTD., Tokyo 102-8667 (JP)
(72) Inventor: KOGA, Kazumi, c/o Banyu Pharmaceutical Co. Ltd., Tsukuba-shi, Ibaraki 300-2611 (JP); OZAKI, Satoshi, c/o Banyu Pharmaceutical Co. Ltd., Tsukuba-shi, Ibaraki 300-2611 (JP); ICHIKAWA, Daisuke, c/o Banyu Pharaceutical Co. Ltd, Tsukuba-shi, Ibaraki 300-2611 (JP); NAMBU, Hirohide, c/o Pharmaceutical Co. Ltd., Tsukuba-shi, Ibaraki 300-2611 (JP); AZUMA, Tomoko, c/o Banyu Pharmaceutical Co. Ltd., Tsukuba-shi, Ibaraki 300-2611 (JP); SAKAI, Naoko, c/o Banyu Pharmaceutical Co. Ltd., Tsukuba-shi, Ibaraki 300-2611 (JP); KAWAGOE, Hiroko, c/o Banyu Pharmaceutical Co. Ltd., Tsukuba-shi, Ibaraki 300-2611 (JP); OHTA, Hisashi, c/o Banyu Pharmaceutical Co. Ltd., Tsukuba-shi, Ibaraki 300-2611 (JP)
(74) Representative: Rollins, Anthony John
(86) International application number: PCT/JP2004/016552
(87) International publication number: WO 2005/045042

(57) **Abstract**

There are provided a non-human primate ORL1 gene etc., using a nucleic acid having the nucleotide sequence listed as SEQ ID NO: 1, a protein having the amino acid sequence listed as SEQ ID NO: 2, a recombinant vector containing a gene consisting of the nucleic acid, and a transformant cell containing the recombinant vector, whereby it is possible to evaluate and screen for compounds that act on ORL1.

## Description

### Technical Field

The present invention relates to a novel simian ORL1 (opioid receptor-like 1) gene and ORL1 protein, a recombinant vector containing the gene, a transformant containing the recombinant vector and a compound evaluation method using the gene or protein.

### Background Art

Nociceptin (also known as "orphanin FQ") is a peptide composed of 17 amino acids having a structure similar to opioid peptides. Nociceptin potentiates reactivity to a noxious stimulus, stimulates appetite, impairs spatial learning ability, antagonizes the analgesic actions of classical opiate agonists, inhibits dopamine release and produces water diuretic effects, vasodilating effects and systemic hypotensive effects, playing a role in pain and appetite regulation as well as memory and learning via ORL1 nociceptin receptors in the brain (Non-patent documents 1, 2, 3, 4, 5 and 6).

ORL1 expression-blocked knockout mice are known to exhibit reduced morphine resistance and improved memory and learning ability (Non-patent documents 7 and 8).

Substances that specifically inhibit binding of nociceptin to ORL1 are therefore expected to be useful as analgesics for painful conditions such as cancer pain, postoperative pain, migraine, gout, chronic rheumatism, chronic pain and neuralgia, as agents used to overcome resistance to narcotic analgesics such as morphine, as agents used to overcome dependency on narcotic analgesics such as morphine, as analgesic action potentiators, as anti-obesity agents, as brain function ameliorators, as therapeutic agents for Alzheimer's disease, as anti-dementia agents, as therapeutic agents for schizophrenia, as therapeutic agents for neurodegenerative diseases such as Parkinson's disease and chorea, as antidepressants, as therapeutic agents for diabetes insipidus, as therapeutic agents for polyuria and as therapeutic agents for hypotension.

Human ORL1 was cloned as an orphan receptor with high homology to opioid receptors, during the course of cloning of opioid receptors (Non-patent document 9). This receptor had not been shown to bind to conventional opioid ligands and its function was unknown, but later experiments reacting brain peptide fraction with cells expressing ORL1 cDNA led to the identification and isolation of nociceptin (or orphanin FQ) as an endogenous ligand with cAMP-decreasing activity (Non-patent documents 10 and 11).

The physiological actions of candidate compounds developed for therapeutic or diagnostic agents are evaluated in rodents and primates. This is because candidate compounds often exhibit different drug effects depending on the animal species, and evaluation in primates, which are more closely related to humans, can lead to development of more effective therapeutic and diagnostic agents.
Non-patent document 1: Nature, Vol. 377, 532 (1995)
Non-patent document 2: Society for Neuroscience, Vol. 22, 455 (1996)
Non-patent document 3: Neuroreport, Vol. 8, 423 (1997)
Non-patent document 4: Eur. J. Neuroscience, Vol. 9, 194 (1997)
Non-patent document 5: Neuroscience, Vol. 75, 1 and 333 (1996)
Non-patent document 6: Life Sciences, Vol. 60, PL15 and PL141 (1997)
Non-patent document 7: Neuroscience Letters, Vol. 237, 136 (1997)
Non-patent document 8: Nature, Vol. 394, 577 (1998)
Non-patent document 9: FEBS Letters, Vol. 341, 33 (1994)
Non-patent document 10: Nature, Vol. 377, 532 (1995)
Non-patent document 11: Science, Vol. 270, 792 (1995)

### Disclosure of the Invention

### Problem to be Solved by the Invention

Nevertheless, the ORL1 genes of primates other than humans have not yet been isolated, and therefore evaluation of therapeutic or diagnostic agents using the genes of non-human primates is still not possible.

It is an object of the present invention, which has been accomplished in light of the aforementioned problems of the prior art, to provide the ORL1 gene and ORL1 protein of a non-human primate. It is another object of the invention to provide a compound evaluation method using the gene and protein.

### Means for Solving the Problem

As a result of much diligent research directed toward achieving the objects stated above, the present inventors succeeded in isolating the rhesus monkey ORL1 gene and evaluating ligands by binding assays using the receptor (rhesus monkey ORL1), and have thereupon completed this invention.

Specifically, a nucleic acid of the invention is characterized by containing the nucleotide sequence listed as SEQ ID NO: 1.

A nucleic acid of the invention is also characterized by consisting of the nucleotide sequence listed as SEQ ID NO: 1. A nucleic acid of the invention is further characterized by coding for a protein consisting of the amino acid sequence listed as SEQ ID NO: 2. A simian ORL1 gene (simian gene coding for a protein having ORL1 activity) consisting of any of these nucleic acid is also provided according to the invention.

A nucleic acid of the invention is further characterized by being a simian isolated nucleic acid which hybridizes under stringent conditions with a nucleic acid consisting of the nucleotide sequence listed as SEQ ID NO: 1 or a nucleotide sequence complementary thereto and which codes for ORL1 protein (protein having ORL1 activity). A simian ORL1 gene consisting of this nucleic acid is also provided according to the invention.

A recombinant vector of the invention is characterized by comprising the aforementioned nucleic acid or gene. A transformant cell of the invention is characterized by comprising the aforementioned recombinant vector.

A protein of the invention is characterized by containing the amino acid sequence listed as SEQ ID NO: 2. A protein of the invention is also characterized by consisting of the amino acid sequence listed as SEQ ID NO: 2. A protein of the invention is further characterized by being an isolated protein consisting of the amino acid sequence listed as SEQ ID NO: 2 with a substitution, deletion, addition or insertion of one or more amino acids, and having ORL1 activity.

By utilizing such a nucleic acid, gene, protein, expression vector or host cell it is possible to construct an expression system for a primate ORL1 or a mutant thereof, and to construct a compound evaluation system using the expression system.

A compound evaluation method of the invention is characterized by comprising a step of transferring a simian ORL1 gene into a cell to prepare a cell expressing the gene, a step of contacting a test compound with the cell, and a step of detecting specific binding of the test compound to a protein (simian protein having ORL1 activity) obtained by expression of the gene.

A compound evaluation method of the invention is further characterized by comprising a step of transferring a simian ORL1 gene into a cell to prepare a cell expressing the gene, a step of contacting a test compound with the cell, a step of assaying the activity of an intracellular signal transducer produced by the contact between the cell and the test compound, and a step of comparing the activity with the activity of the intracellular signal transducer without contact with the test compound.

A compound evaluation method of the invention is still further characterized by comprising a step of contacting a test compound with a simian ORL1 protein (simian protein having ORL1 activity) and a step of detecting a change in activity of the ORL1 protein caused by the contact between the ORL1 protein and the test compound.

These compound evaluation methods allow evaluation and screening of compounds which are candidates for therapeutic and diagnostic agents using a primate ORL1, thereby permitting development of effective drugs in a model system more closely resembling that of humans.

### Effects of the Invention

With the nucleic acid, protein and compound evaluation method of the invention, it is possible to obtain an ORL1 gene and ORL1 protein of a non-human primate, and to evaluate and screen compounds using the gene or protein. The gene or protein may therefore be used for evaluation and development of therapeutic and diagnostic agents.

### Brief Description of the Drawings

Fig. 1 is a diagram showing a homology comparison of the rhesus monkey and human (homo sapiens) ORL1 protein amino acid sequences.
Fig. 2 is a graph showing the relationship between [¹²⁵I][Tyr¹⁴]nociceptin concentration and specific binding to ORL1, in a binding assay using rhesus monkey ORL1.
Fig. 3 is a graph showing the relationship between [¹²⁵I][Tyr¹⁴]nociceptin concentration and specific binding to ORL1, in a binding assay using human ORL1.
Fig. 4 is a graph showing the relationship between nociceptin concentration and specific binding of [³⁵S]GTPγS to ORL1, in a binding assay using rhesus monkey or human ORL1.

### Best Modes for Carrying Out the Invention

Preferred embodiments of the invention will now be explained in detail.

"Nucleic acid" according to the invention refers to, for example, DNA, RNA or modified DNA or RNA, and preferably it is DNA. The DNA may be either genomic DNA or cDNA, and either single-stranded or double-stranded.

An "isolated" nucleic acid or protein according to the invention refers to nucleic acid or protein which is substantially free of cellular substances and culturing medium when produced by a recombinant DNA technique, or substantially free of precursor substances or other substances when produced by chemical synthesis.

The phrase "hybridizes under stringent conditions" according to the invention means that two nucleic acid fragments hybridize with each other under the hybridization conditions described in Molecular Cloning: A Laboratory Manual, 2nd Edition, Cold Spring Harbor (1989), 9.47-9.62 and 11.45-11.61. More specifically, there may be mentioned conditions wherein, for example, hybridization is conducted at about 45°C, 6.0 x SSC and followed by rinsing at 50°C, 2.0 x SSC. For selection of stringency, the salt concentration for the rinsing step may be selected from about 2.0 x SSC, 50°C for low stringency to about 0.2 x SSC, 50°C for high stringency. The temperature for the rinsing step may be from room temperature (about 22°C) for low stringency conditions to about 65°C for high stringency conditions.

Rhesus monkey ORL1 according to the invention will now be explained.

The nucleotide sequence of the rhesus monkey ORL1 gene of the invention has an open reading frame of 1113 nucleotides (SEQ ID NO: 1), coding for a protein of 370 amino acids (SEQ ID NO: 2).

There are no particular restrictions on the method of cloning the rhesus monkey ORL1 gene, and as a specific example there may be mentioned a method wherein a nucleic acid fragment from the rhesus monkey ORL1 gene or a gene having high homology thereto is used for amplification of the full length cDNA by 5'-RACE or 3'-RACE, or a method wherein a cDNA library constructed using an appropriate vector (plasmid vector, bacteriophage, etc.) is screened using a nucleic acid fragment from the rhesus monkey ORL1 gene.

The homology between the rhesus monkey ORL1 gene and the human ORL1 gene is approximately 95.9% in the coding region, with a difference of 44 nucleotides. Nucleic acids of the invention include a nucleic acid with the nucleotide sequence of SEQ ID NO: 1 having a substitution, deletion, insertion or addition of one or more nucleotides. The number of the substituted, deleted, inserted or added nucleotides is preferably 1-43 nucleotides, more preferably 1-30 nucleotides, even more preferably 1-20 nucleotides, still more preferably 1-10 nucleotides and yet still more preferably 1-5 nucleotides. The nucleic acid preferably has at least 96% homology, more preferably at least 97% homology, even more preferably at least 98% homology and most preferably at least 99% homology to the nucleotide sequence listed as SEQ ID NO: 1. The nucleic acid may be obtained by isolating a nucleic acid of the invention which hybridizes under stringent conditions with a nucleic acid consisting of the nucleotide sequence listed as SEQ ID NO: 1 or a nucleotide sequence that is complementary thereto. There are no particular restrictions on the species from which the nucleic acid is derived, although specifically a simian ORL1 gene (gene coding for a protein with ORL1 activity) is preferred, with examples of simian ORL1 genes including ORL1 genes of rhesus monkey, cynomolgus monkey, Japanese monkey, squirrel monkey, green monkey, anubis baboon and common marmoset.

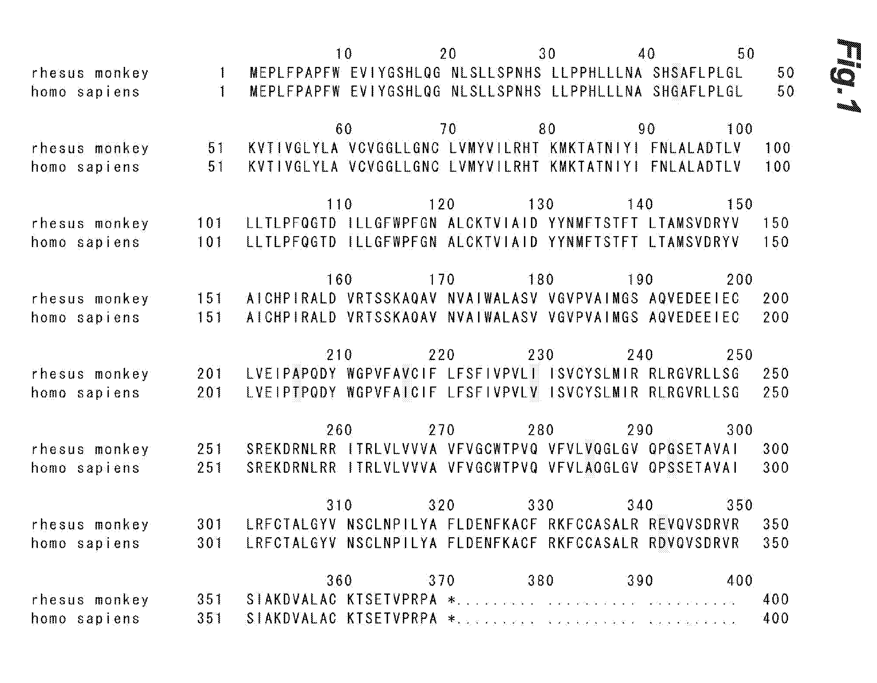

A protein having the amino acid sequence listed as SEQ ID NO: 2 (rhesus monkey ORL1 protein) consists of 370 amino acids, as mentioned above. As shown in Fig. 1, the homology between the rhesus monkey ORL1 protein (SEQ ID NO: 2) and the human ORL1 protein (SEQ ID NO: 3) is 97.8%, with a difference of 7 amino acids.

The method of preparing the rhesus monkey ORL1 is not particularly restricted, and specifically there may be mentioned a method wherein an isolated rhesus monkey ORL1 gene is inserted into an expression vector, the expression vector (recombinant vector) is transferred into host cells such as animal cells, insect cells or *E. coli* cells for expression of the gene, and the protein (gene product) is purified.

The invention encompasses a protein having the amino acid sequence listed as SEQ ID NO: 2 or a mutant thereof. As such mutants there may be mentioned proteins consisting of the amino acid sequence listed as SEQ ID NO: 2 with a substitution, deletion or insertion of one or between 2 and 6 amino acids, and proteins consisting of the same amino acid sequence with an addition of one or more amino acids. Such mutant proteins are preferably biologically active, and more preferably have ORL1 activity.

The invention further provides an expression vector (recombinant vector) containing any one of the following nucleic acids or genes.
1. An isolated nucleic acid comprising the nucleotide sequence listed as SEQ ID NO: 1.
2. An isolated nucleic acid consisting of the nucleotide sequence listed as SEQ ID NO: 1, or a simian ORL1 gene consisting of this nucleic acid.
3. An isolated nucleic acid coding for a protein consisting of the amino acid sequence listed as SEQ ID NO: 2, or a simian ORL1 gene consisting of this nucleic acid.
4. A simian isolated nucleic acid which hybridizes under stringent conditions with a nucleic acid consisting of the nucleotide sequence listed as SEQ ID NO: 1 or a nucleotide sequence complementary thereto and which codes for a protein having ORL1 activity, or a simian ORL1 gene consisting of this nucleic acid.

The recombinant vector of the invention may be obtained by inserting the nucleic acid or gene of the invention into an appropriate expression vector by a publicly known method. The expression vector used is not particularly restricted so long as it is well known to those skilled in the art, and as examples there may be mentioned pcDNA3.1, pBlueBacHis2, pCI-neo, pcDNAI, pMClneo, pXT1, pSG5, pEF1/V5-HisB, pCR2.1, pET11, λgt11 and pCR3.1. The recombinant vector of the invention is preferably one that is capable of autonomous replication in host cells, and contains a promoter, a ribosome binding sequence (SD sequence) and a terminator.

The phrase "comprising the nucleotide sequence listed as SEQ ID NO: 1" in 1. above means that there may be added to the aforementioned nucleotide sequence a vector linker sequence, or a sequence necessary for modification of the gene, such as a restriction endonuclease cleavage site used for gene recombination.

The invention further provides a host cell (transformant cell) containing the aforementioned recombinant vector.

The transformant cell of the invention may be obtained by transferring a recombinant vector of the invention into suitable host cells by a publicly known method. The host cell used is not particularly restricted so long as it is known to those skilled in the art, and may be an animal cell, insect cell, plant cell or microbe. Specifically, as examples there may be mentioned COS1, COS7, CHO, NIH/3T3, 293, Raji, CV11, C1271, MRC-5, CPAE, L-M(TK-), HeLa, 293T and Sf9. The method of transferring the recombinant vector into host cells is not particularly restricted so long as it is a publicly known method, and specifically there may be mentioned electroporation, the calcium phosphate method, the DEAE-dextran method, lipofection and the gene gun method.

The invention also encompasses an antibody for the protein having the amino acid sequence listed as SEQ ID NO: 2 and a mutant thereof. The antibody may be prepared using the protein or a partial peptide thereof as an antigen, and may be monoclonal or polyclonal antibody. The antibody may react not only with simian ORL1 proteins but also with ORL1 proteins of other species depending on the amino acid sequence of the epitope, but for preparation of an antibody that is selective only for simian ORL1 proteins, an antibody with the desired specificity can be prepared by creating a monoclonal antibody using as a peptide antigen a region of a simian ORL1 protein which has low homology to ORL1 proteins of other species.

Uses of the nucleic acid having the nucleotide sequence listed as SEQ ID NO: 1 and the protein having the amino acid sequence listed as SEQ ID NO: 2 according to the invention will now be explained.

### (1) Evaluation of compounds

The nucleic acid or protein of the invention may be used to evaluate compounds that act on ORL1. The method of detecting action on ORL1 may be a method wherein specific binding of a test compound to the receptor is detected, a method wherein a gene expression level which is altered by contact with the test compound is detected, or a method wherein the activity of intracellular signal transduction via ORL1 by the contact is detected. This will be explained in order below.

First, a method of evaluating a test compound by detecting specific binding of the test compound to the receptor will be explained.

A compound evaluation method of the invention is characterized by comprising a step of transferring a simian ORL1 gene into a cell to prepare a cell expressing the gene, a step of contacting a test compound with the cell, and a step of detecting specific binding of the test compound to a protein (simian ORL1 protein) obtained by expression of the gene.

A second compound evaluation method of the invention is characterized by comprising a step of transferring a simian ORL1 gene into a cell to prepare a cell expressing the gene, a step of contacting a test compound with the cell, a step of assaying the activity of an intracellular signal transducer produced by the contact between the cell and the test compound, and a step of comparing the activity with the activity of the intracellular signal transducer without contact with the test compound.

The test compound is not particularly restricted, and as examples there may be mentioned proteins, peptides, non-peptide compounds and artificially synthesized compounds.

The cells expressing the simian ORL1 gene may be prepared by a protocol that is publicly known to those skilled in the art, and while there are no particular restrictions on the specific method employed, the following may be described as an example. First, a nucleic acid having the nucleotide sequence listed as SEQ ID NO: 1 or comprising a portion thereof according to the invention is cloned into an expression vector containing a suitable promoter and a suitable transcriptional control element. The vector is not particularly restricted so long as it can be used as an expression vector, and as examples there may be mentioned pcDNA3.1, pBlueBacHis2, pCI-neo, pcDNAI, pMC1neo, pXT1, pSG5, pEF1/V5-HisB, pCR2.1, pET11, λgt11 and pCR3.1.

Next, the expression vector (recombinant vector) having the nucleic acid of the invention inserted therein is transferred into host cells. The host cell is not particularly restricted so long as it is commonly used for gene expression, and may be an animal cell, insect cell, plant cell or microbe, specific examples of which include COS1, COS7, CHO, NIH/3T3, 293, Raji, CV11, C1271, MRC-5, CPAE, HeLa, 293T and Sf9. The method of transferring the recombinant vector into host cells is not particularly restricted so long as it is a publicly known method, and specifically there may be mentioned electroporation, the calcium phosphate method, the DEAE-dextran method, lipofection and the gene gun method.

The test compound is then contacted with the cells (transformant cells) expressing the ORL1 gene prepared in the manner described above. The method of contact is not particularly restricted, and for example, there may be mentioned contact in a solution such as an aqueous solution or buffer solution.

Binding of the test compound with receptors expressed on the cell surfaces may for example be detected with a label attached to the bound compound (for example, by radioactivity or fluorescent intensity), or based on signal transduction in cells by binding of the test compound to the cell surface receptors (for example, using G protein activation, variations in Ca²⁺ or cAMP concentration, phospholipase C activation, pH variation or receptor internalization). The expression level and activity of molecules in the signal transduction pathway which are produced by the signal transduction may also be the basis of detection. When detection is based on the expression level, there are no particular restrictions on the method of assaying the expression level, and for example, Northern blotting, Western blotting or a DNA chip may be employed. The term "expression level" according to the invention means the absolute or relative level of transcript (mRNA) or translation product (protein) of the gene (DNA or mRNA) coding for a protein in the ORL1-mediated signal transduction pathway. When detection is based on the activity of a molecule in the signal transduction pathway, the activity assay method is not particularly restricted and may be selected from among suitable methods for the type of molecule to be assayed.

On the other hand, an isolated simian ORL1 protein (simian protein having ORL1 activity) may also be used directly for evaluation of compounds. Spacifically, there may be employed a method wherein a test compound is contacted with a simian ORL1 protein and then a change in activity of the ORL1 protein resulting from the contact is detected.

The method of the contact is not particularly restricted, and as specific examples there may be mentioned a method wherein contact is carried out by admixture in a buffer solution (phosphate buffer or the like) or other solution, or a method wherein the ORL1 protein is immobilized on a membrane and contacted with the test compound on the membrane.

The change in activity of the ORL1 protein resulting from the contact is then detected.

The method of detecting a change in activity of the protein may be appropriately set depending on the nature of the protein used, and as a specific example there may be mentioned a method based on the binding activity of nociceptin for ORL1.

There are no particular restrictions on a method based on the binding activity of nociceptin, and specifically there may be mentioned a method wherein binding activity is assayed by measuring the affinity of a test compound for a membrane on which the ORL1 protein is immobilized. The test compound used here may be labeled with a radioactive isotope etc. for easier detection. The method employed for detection of binding activity may be a method wherein a compound which binds to ORL1 competitively against nociceptin that has been labeled with a radioactive isotope is detected, which method requires no labeling of the test compound.

Upon detection of the compound by the compound evaluation method described above, if the binding activity of nociceptin in the presence of the test compound is a lower value than the binding activity of nociceptin in the absence of the test compound (control), the test compound is judged as inhibiting binding of ORL1 to its ligand or an analog thereof. Such compounds include those with activity of inducing signal transduction in cells upon binding to the receptor (ORL1) (agonists) and those without such activity (antagonists). Agonists have the same bioactivity as ligands for ORL1 and analogs thereof. Antagonists, on the other hand, do not have the bioactivity of ligands for ORL1 and analogs thereof while inhibiting binding of ligands for ORL1 and analogs thereof to ORL1. Consequently, such agonists and antagonists are useful in pharmaceutical compositions for treatment of diseases associated with defective signal transduction via ORL1.

The compound evaluation method of the invention allows screening of substances that promote or inhibit intracellular signal transduction after binding of a test compound to ORL1. That is, by evaluating different test compounds by the method described above it is possible to select compounds that function as agonists or antagonists. If, as a result of selection, change in intracellular signal transduction is suppressed compared to the change with nociceptin or its analog in the absence of the test compound, then the test compound is judged to be a compound that inhibits intracellular signal transduction upon binding of the test compound to ORL1. Conversely, if the test compound augments intracellular signal transduction, then the compound is judged as being a compound that promotes intracellular signal transduction upon binding of the test compound to ORL1. Compounds selected by this screening method are expected to be useful as drugs for various conditions associated with nociceptin and ORL1, for example, as analgesics for painful conditions such as cancer pain, postoperative pain, migraine, gout, chronic rheumatism, chronic pain and neuralgia, as agents used to overcome resistance to narcotic analgesics such as morphine, as agents used to overcome dependency on narcotic analgesics such as morphine, as analgesic action potentiators, as anti-obesity agents, as brain function ameliorators, as therapeutic agents for Alzheimer's disease, as anti-dementia agents, as therapeutic agents for schizophrenia, as therapeutic agents for neurodegenerative diseases such as Parkinson's disease and chorea, as antidepressants, as therapeutic agents for diabetes insipidus, as therapeutic agents for polyuria and as therapeutic agents for hypotension.

The compound evaluation method of the invention as described above also allows evaluation of ligands used for PET (Positron Emission Tomography). PET is a non-invasive method for observing biological function by labeling a substance found in the body such as water, oxygen, glucose or an amino acid, or a drug (ligand for a target receptor, for example) with a radioactive isotope and administering it to the body, and the method is used for research and in clinical practice. PET allows function-specific imaging dependent on the ligand used as the tracer, and development of new tracers is indispensable for elucidation of unknown biological functions and diagnosis of disease. In the compound evaluation method of the present invention, the PET ligand candidate substance may be used as the test compound to allow *in vitro* evaluation of the substance.

### (2) Probe used for hybridization

The ORL1 gene can be detected by using a nucleic acid consisting of all or a portion of the nucleotide sequence listed as SEQ ID NO: 1 of the invention as a hybridization probe. The detection is not limited to detection of the ORL1 gene in a specimen of biological tissue or cells, but may also be applied for cloning of the ORL1 gene or a gene having high homology to the gene. Also, the nucleic acid may be used as a probe for identification of gene expression distribution by examining gene expression in different tissues.

When the nucleic acid is used as a probe, there are no particular restrictions on the method of hybridization, and as examples there may be mentioned Southern hybridization, Northern hybridization, colony hybridization, dot hybridization, fluorescence in situ hybridization (FISH), in situ hybridization (ISH) and the DNA chip method.

When the nucleic acid is used as a probe for hybridization, a nucleic acid with a length of at least 20 contiguous nucleotides is required, and the length is preferably at least 40 nucleotides, more preferably at least 60 nucleotides and most preferably at least 80 nucleotides. The probe may also be labeled to allow detection if necessary. Specifically, it may be labeled with a radioactive isotope such as ³²P, ¹⁴C, ¹²⁵I, ³H or ³⁵S, or it may be labeled with biotin, a fluorescent dye, an enzyme, gold colloid or the like.

The conditions for hybridization when the nucleic acid is used as a probe for hybridization may be selected as appropriate by a person skilled in the art depending on the length of the probe, and the type of gene which is the target of hybridization. It may be carried out, for example, with reference to Molecular Cloning: A Laboratory Manual, 2nd Edition, Cold Spring Harbor (1989), 9.47-9.62 and 11.45-11.61. More specifically, there may be mentioned conditions wherein hybridization is conducted at about 45°C, 6.0 x SSC and followed by rinsing at 50°C, 2.0 x SSC. For selection of stringency, the salt concentration for the rinsing step may be selected from about 2.0 x SSC, 50°C for low stringency to about 0.2 x SSC, 50°C for high stringency. The temperature for the rinsing step may be from room temperature (about 22°C) for low stringency conditions to about 65°C for high stringency conditions.

### (3) Primers used for PCR

The method of detecting the ORL1 gene may be the Polymerase Chain Reaction (PCR) using portions of the nucleotide sequence listed as SEQ ID NO: 1 of the invention as primers.

The lengths of the primers used may be appropriately selected based on their nucleotide sequences and the nucleotide sequence of a gene to be isolated, but generally, they will be preferably 10-60, more preferably 15-30, contiguous nucleotides.

### Examples

The present invention will now be explained in greater detail through examples, with the understanding that these examples are in no way limitative on the invention.

(Example 1: Isolation of rhesus monkey ORL1 cDNA).
Total RNA was prepared from rhesus monkey prefrontal cortex using ISOGEN (Nippon Gene). An oligo dT primer and AMV transcriptase (Life Science) were used to synthesize 1st strand cDNA from the total RNA. Primers designed for the 5' and 3' untranslated regions of human ORL1 (5'-TACCGTACAGAGTGGATTTGC (SEQ ID NO: 3) and 3'-ACGGGTACCACGGACAG (SEQ ID NO: 4)) were used for amplification of the full length ORL1 from the rhesus monkey prefrontal cortex cDNA. The gene was amplified using TaKaRa ExTaq (Takara Shuzo), with 30 cycles of 94°C for 45 sec, 50°C for 72 sec and 72°C for 90 sec. The 1.2 kb nucleic acid fragment amplified in this manner was isolated, and subcloned into an expression vector pCR3.1 using a Eukayrotic TA Cloning Kit (Invitrogen). Primers designed for different locations on rhesus monkey ORL1 were used to determine the nucleotide sequences of both strands using a sequencer. The nucleotide sequences were confirmed by determining the nucleotide sequences of multiple clones.

(Example 2: Preparation of cells stably expressing rhesus monkey ORL1)
CHO (Chinese Hamster Ovary)-K1 cells were transfected with an expression vector (recombinant vector) containing the full-length rhesus monkey ORL1 using Transfectam Reagent (Promega). The cells were cultured at 37°C in the presence of 5% CO₂ using Ham's F12 medium containing 10% fetal calf serum and 1 mg/ml geneticin (Gibco). Cells exhibiting geneticin resistance were cloned, and the ORL1-expressing clones (CHO-rhORL1) were identified by binding assay using [¹²⁵I][Tyr¹⁴]nociceptin.

(Example 3)
Preparation of membrane:
First, CHO-rhORL1 cells were homogenized in 10 mM 3-(N-morpholino)propanesulfonic acid (MOPS) buffer (pH 7.4) containing 154 mM NaCl, 10 mM KCl, 0.8 mM CaCl₂ and 20% sucrose. The homogenate was centrifuged at 10,000 g and the supernatant was subsequently centrifuged at 100,000 g. The obtained precipitate was homogenized in 5 mM HEPES/Tris (pH 7.4). The protein concentration was measured using a BCA protein assay kit (Sigma) with BSA as control.

Radioligand binding assay:
The membrane was incubated for 60 minutes at 37°C, pH 7.4 together with a total of 200 µL of solution containing different concentrations (10-320 pM) of [¹²⁵I][Tyr¹⁴]nociceptin, 50 mM HEPES, 10 mM NaCl, 1 mM MgCl₂, 2.5 mM CaCl₂, 0.1% bovine serum albumin (BSA) and 0.025% bacitracin. The membrane was then incubated in the presence of 1 µmol/l of unlabeled nociceptin, the total residual radioactivity was recorded as the non-specific binding, and this was subtracted from the measured value to obtain the specific binding. The incubation was terminated by rapidly passing through a GF/C filter pre-immersed in 0.5% polyethyleneimine, using a cell harvester. The filter was then rinsed three times at 4°C with 5 mM HEPES/Tris, 0.1 % BSA (pH 7.4). The residual radioactivity on the filter was measured using a gamma counter (Packard). The results were analyzed using a Prism Software Package (Graphpad).

(Comparative Example 1)
Analysis was conducted in the same manner as Example 3, except that the human ORL1 gene was used instead of the rhesus monkey ORL1 gene.

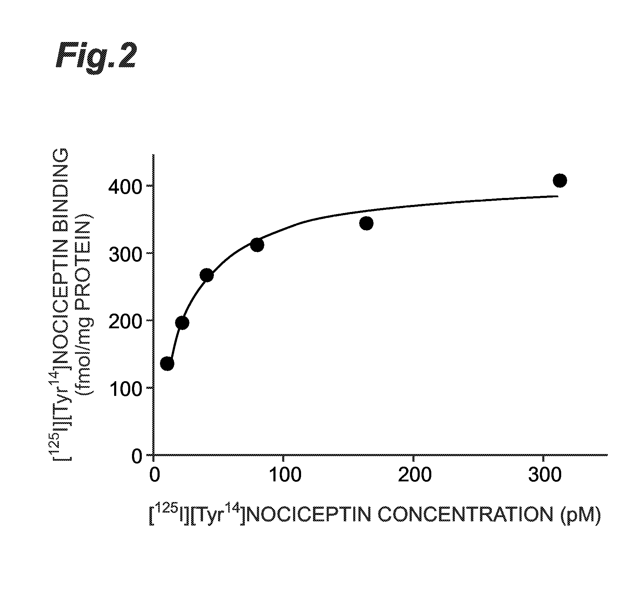

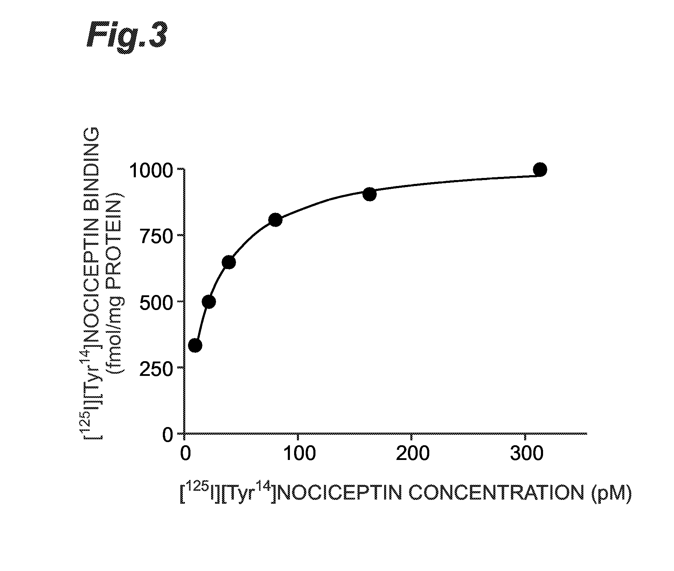

The results of Example 3 and Comparative Example 1 (Figs. 2 and 3) showed that the dissociation constant (kd) of [¹²⁵I][Tyr¹⁴]nociceptin for rhesus monkey ORL1 is 27 ±4 pM, demonstrating that nociceptin has the same high affmity for simian ORL1 as for human ORL 1.

### (Example 4: [³⁵S]GTPγS binding assay)

The membrane prepared in Example 3 was incubated together with 200 pM [³⁵S]GTPγS in a solution at pH 7.4 containing 20 mM HEPES, 100 mM NaCl, 10 mM MgCl₂, 1 mM EDTA and 5 µM GDP. SPA beads coated with wheat germ agglutinin were added to the solution, and the incubation was performed at 25°C for 2.5 hours in the presence of different concentrations of nociceptin and in the absence of of nociceptin. The membrane was then incubated in the presence of unlabeled GTPγS (10 µmol/l), the total residual radioactivity was recorded as the non-specific binding, and this was subtracted from the measured value to obtain the specific binding. The radioactivity bound to the membrane was detected using a TopCount microplate scintillation counter (Packard).

### (Comparative Example 2)

Analysis was conducted in the same manner as Example 4, except that the human ORL1 gene was used instead of the rhesus monkey ORL1 gene.

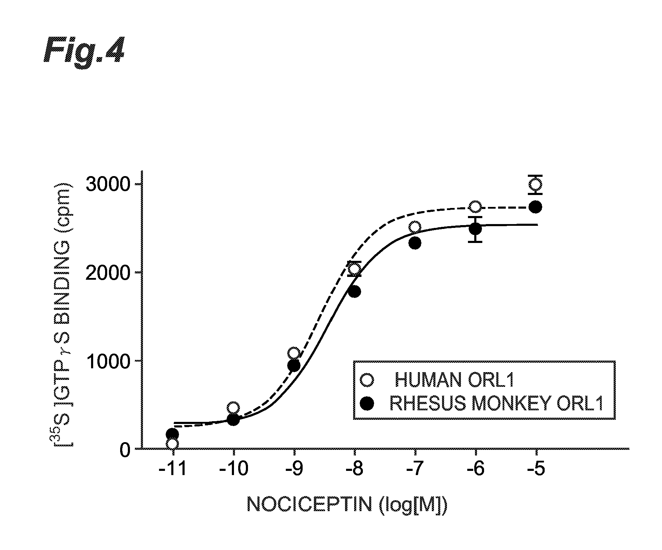

The results of Example 4 and Comparative Example 2 (nociceptin dose response curves shown in Fig. 4) showed that the EC₅₀ for rhesus monkey ORL1 is 2.3 ±0.4 nM, confirming that simian ORL1 has the same function for nociceptin as does human ORL1.

### Industrial Applicability

According to the nucleic acid, protein and compound evaluation method of the invention, it is possible to obtain an ORL1 gene and ORL1 protein of a non-human primate, and compound evaluation and screening can be carried out using the gene or protein. Thus, therapeutic and diagnostic agents can be evaluated and developed using the gene or protein. A major contribution is therefore provided for increased efficiency of research and development of human drugs.

## Claims

1. An isolated nucleic acid consisting of the nucleotide sequence listed as SEQ ID NO: 1.

2. An isolated nucleic acid coding for a protein consisting of the amino acid sequence listed as SEQ ID NO: 2.

3. A isolated simian nucleic acid which hybridizes under stringent conditions with a nucleic acid consisting of the nucleotide sequence listed as SEQ ID NO: 1 or a nucleotide sequence complementary thereto and which codes for a protein having ORL1 activity.

4. A simian ORL1 gene consisting of the nucleic acid according to any one of claims 1 to 3.

5. A recombinant vector containing the simian ORL1 gene according to claim 4.

6. A transformed cell containing the recombinant vector according to claim 5.

7. An isolated protein consisting of the amino acid sequence listed as SEQ ID NO: 2.

8. An isolated protein consisting of the amino acid sequence listed as SEQ ID NO: 2 with the substitution, deletion, addition or insertion of one or more amino acids, and having ORL1 activity.

9. A compound evaluation method comprising:
a step of transferring a simian ORL1 gene into a cell to prepare a cell expressing the gene,
a step of contacting a test compound with the cell, and
a step of detecting specific binding of the test compound to a protein obtained by expression of the gene.

10. A compound evaluation method comprising:
a step of transferring a simian ORL1 gene into a cell to prepare a cell expressing the gene,
a step of contacting a test compound with the cell,
a step of assaying the activity of an intracellular signal transducer produced by the contact between the cell and the test compound, and
a step of comparing the activity with the activity of the intracellular signal transducer without contact with the test compound.

11. A compound evaluation method comprising:
a step of contacting a test compound with a simian protein having ORL1 activity, and
a step of detecting a change in activity of the protein caused by the contact between the protein and the test compound.

12. The compound evaluation method according to any one of claims 9 to 11, wherein the simian is a rhesus monkey.
